# EUROPEAN PATENT APPLICATION

(11) **EP 3 950 961 A1**
(43) Date of publication of application: **09.02.2022**
(21) Application number: 20777844.0
(22) Date of filing: 23.03.2020
(51) Int. Cl.: C12Q 1/6886, A61K 31/47, A61P 35/00

(54) **APPLICATION OF KDM5A GENE AND ATRX GENE**

(30) Priority: 25.03.2019 CN 201910228411
(71) Applicant: Shenzhen Chipscreen Biosciences Co., Ltd., Shenzhen, Guangdong 518057 (CN)
(72) Inventor: LU, Xianping, Shenzhen, Guangdong 518057 (CN); SHAN, Song, Shenzhen, Guangdong 518057 (CN); PAN, Desi, Shenzhen, Guangdong 518057 (CN); NING, Zhiqiang, Shenzhen, Guangdong 518057 (CN)
(74) Representative: J A Kemp LLP
(86) International application number: PCT/CN2020/080579
(87) International publication number: WO 2020/192606

(57) **Abstract**

The present invention provides use of KDM5A gene and/or ATRX gene as biomarkers in evaluating the efficacy of Chiauranib or guiding the administration of Chiauranib, and use of Chiauranib for the manufacture of a drug for treating small cell lung cancer patients with gene mutations in KDM5A gene or ATRX gene.

## Description

The present application claims the priority of a Chinese patent application filed with the Chinese Patent Office on March 25, 2019, with an application number of 201910228411.9 and an invention title of "Use of KDM5A Gene and ATRX Gene", the entire content of which is incorporated herein by reference.

### TECHNICAL FIELD

The present invention relates to the technical field of drugs, and particularly relates to use of KDM5A gene and ATRX gene.

### BACKGROUND ART

Lung cancer ranks first among malignant tumors in regards to morbidity and mortality. Small cell lung cancer (SCLC) accounts for 10% to 15% of all lung cancers, and its clinical characteristics and biological behavior are different from other lung cancers, showing short doubling time, early metastasis, and high degree of malignancy. Untreated patients often die within 2 to 4 months. Although newly-treated patients are more sensitive to chemotherapy, they are prone to drug resistance and relapse, and are relatively insensitive to second-line chemotherapy drugs, resulting in poor prognosis. 30% to 40% of the patients diagnosed are in the limited stage, and 60% to 70% of the patients diagnosed are in the extensive stage. The long-term survival rate for patients in the limited stage is 20%, while the long-term survival rate for patients in the extensive stage is 2%.

Etoposide / cisplatin (EP) regimen is currently the main chemotherapy regimen for SCLC. The results of phase III clinical study showed that for patients in the limited-stage SCLC, 2- and 5-year survival rates in the EP regimen were superior to those in a cyclophosphamide / epirubicin / vincristine regimen (25% *νs*. 10%, and 8% *νs*. 3%); and for patients in the extensive-stage SCLC, the EP regimen can also bring survival benefit. A series of subsequent studies have also proved the effectiveness of the EP regimen, so the EP regimen becomes the standard first-line chemotherapy for SCLC.

The results of the irinotecan / cisplatin (CPT-11/DDP, IP) regimen group and the EP regimen group showed that objective response rates (ORR) of patients in the two groups were 84.4% and 67.5% (P = 0.02), respectively, and the median survivals were 12.8 months and 9.4 months (P = 0.002), respectively. Overall survival, quality of life, improvement of symptoms of a topotecan combined optimal supportive treatment group are all significantly better than those of the monotherapy optimal supportive treatment group, so topotecan also becomes a second-line chemotherapy drug for SCLC. In summary, SCLC lacks an effective therapy, and has fewer second-line options (e.g. topotecan and paclitaxel) upon failure of the conventional EP or IP regimen. Moreover, guidelines such as NCCN only recommend supportive treatments or clinical studies upon failure of the second-line therapies. Therefore, it is necessary to explore efficient therapeutic regimens for small cell lung cancers.

### SUMMARY

In view of the above, the present invention is directed to provide use of KDM5A gene and/or ATRX gene as biomarkers in evaluating the efficacy of Chiauranib or guiding the administration of Chiauranib.

A compound with a generic name of Chiauranib is currently in clinical trials, its chemical name is *N*-(2-aminophenyl)-6-(7-methoxyquinolin-4-oxy)-1-naphthamide, and its structural formula is shown as Formula (I):

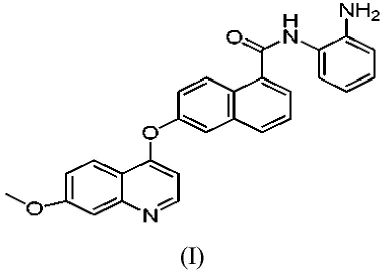

According to the present invention, a phase Ib clinical trial of using Chiauranib capsules to treat relapsed and refractory small cell lung cancer was carried out, and a concomitant study of efficacy-related biomarkers for 548 tumor-related genes was carried out by detecting and analyzing plasma free tumor DNA (ctDNA). Blood samples were taken from all patients before enrollment, and gene sequences of tumor-related genes were detected, including gene mutations and copy number abnormalities. According to detection results, genes with mutation rates of more than 0.4% were all selected, and progression free survival (PFS) and objective response rates (ORR) of patients were taken as efficacy indicators to analyze the correlation between tumor-related gene abnormalities and the efficacy of Chiauranib. Results showed that among the 548 tumor-related genes, only KDM5A gene and ATRX gene had significant correlation with the efficacy of Chiauranib.

Specific test results showed that KDM5A gene and ATRX gene mutations had significant correlation with PFS and ORR, respectively, of Chiauranib in the small cell lung cancer patients. Median PFS was 145 days in patients with KDM5A gene mutation, 27.5 days in wild-type patients, and the P value was 0.0087; when objective response (PR) and non-response (SD+PD) were taken as efficacy evaluation indicators, the efficacy evaluation of patients with ATRX gene mutation was significantly superior to that of the wild-type patients, and the P value was 0.0031.

According to the above technical effects, the present invention further correspondingly provides use of a product for detecting KDM5A gene and/or ATRX gene mutation for the manufacture of a product for evaluating the efficacy of Chiauranib or guiding the administration of Chiauranib; and use of KDM5A gene and/or ATRX gene for the manufacture of a biomarker for evaluating the efficacy of Chiauranib or guiding the administration of Chiauranib.

In addition, the present invention further provides a method of evaluating the efficacy of Chiauranib or guiding the administration of Chiauranib, including: performing gene mutation detection on a tumor tissue of small cell lung cancer, and determining that the efficacy of Chiauranib is better if a gene mutation occurs in KDM5A gene or ATRX gene.

In specific embodiments of the present invention, ctDNA of a tumor tissue of small cell lung cancer is taken as a test sample and detected by next generation sequencing; there are a variety of gene mutation detection methods, which are not limited to the next generation sequencing of ctDNA in the specific embodiments. For samples from tumor tissues, tumor circulating cells or other human sources, other detection methods such as gene sequencing, PCR, FISH and immunohistochemistry can be used to detect gene mutations.

It can be seen from the above technical solutions that the present invention verifies the correlation between the KDM5A gene and ATRX gene mutations and the efficacy of Chiauranib by taking the progression free survival (PFS) and the objective response rates (ORR) of the patients as the efficacy indicators, and the detection of KDM5A gene and ATRX gene mutation information can guide the clinical administration of Chiauranib and evaluate its efficacy on small cell lung cancer, which is particularly suitable for refractory and relapsed small cell lung cancer.

### DETAILED DESCRIPTION OF THE EMBODIMENTS

The present invention discloses use of KDM5A gene and ATRX gene. Those skilled in the art may learn from the contents herein to appropriately modify process parameters to implement the present invention. In particular, it should be pointed out that all similar substitutions and modifications are obvious to those skilled in the art, and they are all deemed to be included in the present invention. The use of the present invention has been described through the preferred embodiments. It is obvious that relevant personnel can make modifications or appropriate changes and combinations to the use described herein without departing from the content, spirit and scope of the present invention to implement and apply the technology of the present invention.

The use of the KDM5A gene and the ATRX gene provided in the present invention will be further described below.
**Embodiment 1**: Phase Ib clinical trial of Chiauranib monotherapy for treating relapsed and refractory small cell lung cancer
**Test drug:** Chiauranib capsules, with specifications of 5 mg and 25 mg. They were manufactured by Shenzhen Chipscreen Biosciences Co., Ltd.
**Dosing regimen:** the Chiauranib capsules were administered QD at 50 mg/day (not adjusted according to the body weight or the body surface area). The capsules were taken on an empty stomach every morning with water, and the whole capsules were swallowed completely. Continuous administration for 28 days was one treatment cycle, and there was no interval during each treatment cycle.
**Number of cases:** 25 patients were enrolled.
**Inclusion criteria:**
   1. Age ≥ 18 years, and ≤ 75 years, with no gender limitation;
   2. Small cell lung cancer was confirmed by histology or cytology;
   3. A progressed or relapsed disease occurred after at least 2 different systemic chemotherapies (including platinum-containing chemotherapy regimens) were received in the past;
   4. According to the RECIST 1.1 criteria, there was at least one measurable target lesion, and the lesions treated by radiotherapy or local area treatment must have imaging evidence of disease progression before they can be regarded as target lesions;
   5. ECOG performance score was 0 to 1;
   6. Major organ functions met the following criteria:
      blood routine: absolute neutrophil count ≥ 1.5×10⁹ /L, platelet count ≥ 75×10⁹ /L, and hemoglobin ≥ 80 g/L;
      blood biochemistry: total bilirubin ≤ 1.5 times the upper limit of normal value, AST/ALT ≤ 2.5 times of the upper limit of normal value (if in the case of hepatic metastasis, ≤ 5 times the upper limit of normal value), and serum creatinine ≤ 1.5 times the upper limit of normal value; and
      coagulation function: prothrombin time-International normalized ratio (PT-INR) < 1.5.
   7. Expected survival time≥ 3 months; and
   8. A written informed consent was voluntarily signed.
**Treatment plan:**
   The test subjects took 50 mg of Chiauranib capsules orally once daily, every 28 days was taken as one treatment cycle, and there was no withdrawal interval during the treatment cycles. All subjects received continuous treatment throughout the trial period until any one of the following occurred (whichever occurred first): progressed disease, intolerable toxicity, death, withdrawal of the informed consent, or loss to follow-up.
**Efficacy evaluation:** according to the RECIST 1.1 criteria, evaluations were respectively performed in the baseline period and at the end of the 4th week after treatment, and repeated every 8 weeks until a progressive disease occurred. Tumor imageological examinations included CT or MRI of neck, chest, whole abdomen, and pelvic cavity. Other site examinations should be performed as necessary according to clinical indications. The same technologies and methods should be used for baseline of lesions and subsequent evaluation and measurement.
**Safety evaluation:** physical examination, vital signs, ECOG performance score, blood routine, urine routine, 12-lead ECG, blood biochemistry, electrolyte, coagulation function, myocardial enzyme, troponin, TSH, FT3, FT4, amylase, echocardiogram, 24-hour urine protein quantification (if necessary), and adverse events were included.
**Biomarker study:**
   10 mL of peripheral blood was taken before the subjects took Chiauranib for the first time and when a progressive disease occurred, gene sequences of plasma free tumor DNA (ctDNA) and leukocyte extracted DNA (control) were detected, a total of 548 tumor-related genes were included, and detection results included gene mutations and copy number abnormalities, as well as analysis of tumor mutation burden (TMB).
**Results of clinical trial:**

25 patients were enrolled, and among the 25 patients, 20 patients were subjected to efficacy evaluation. Among the 20 patients, 4 patients had the best efficacy evaluation being partial response (PR), the ORR was 20%, and the benefit rate was 60%. The results showed that the Chiauranib monotherapy was effective in the treatment of small cell lung cancer.

Plasma free tumor DNA (ctDNA) of the evaluated patients was detected and analyzed, and a concomitant study of efficacy-related biomarkers for the 548 tumor-related genes was carried out. According to detection results, genes with mutation rates of more than 0.4% were all selected, and progression free survival (PFS) and objective response (PR) of the patients were taken as efficacy indicators to analyze the correlation between tumor-related gene abnormalities and the efficacy of Chiauranib. Results showed that among the 548 tumor-related genes, only KDM5A gene and ATRX gene had significant correlation with the efficacy of Chiauranib.

The KDM5A gene had significant correlation with the benefit of progression free survival (PFS) of the patients, and results are shown in Table 1.

**Table 1**

| PFS evaluation | Wild type (*n* = 12) | Mutation (n = 8) |
|---|---|---|
| Median (95% CI) | 27.5 (16.0, 61.0) | 145.0 (23.0, 145.0) |
| Hazard ratio (HR) | 16.5 (2.03, 133.37) | |
| P value | 0.0087 | |

The results in Table 1 showed that when the progression free survival (PFS) of the patients as the efficacy evaluation indicator, the KDM5A gene mutation had significant correlation with the benefit of PFS of the patients. There were 12 patients with wild-type KDM5A gene, and the median PFS was 27.5 days; and there were 8 patients with KDM5A gene mutation, and the median PFS was 145 days. There was a significant statistic difference between the progression free survival of the patients with KDM5A gene mutation and the progression free survival of wild-type patients gene, and the P value was 0.0087. It indicated that the patients with KDM5A gene mutation can obtain better benefit from the Chiauranib therapy.

The ATRX gene had significant correlation with the benefit of objective response (PR) of the patients, and results are shown in Table 2.

**Table 2**

| Efficacy evaluation | Wild type (%) | Mutation (%) | P value |
|---|---|---|---|
| Non-response (SD + PD) | 14 (100.0) | 2 (33.3) | 0.0031 |
| Obj ective response (PR) | 0 | 4 (66.7) | |
| Total | 14 (100.0) | 6 (100.0) | |

The results in Table 2 showed that when the objective response (PR) and non-response (SD+PD) were taken as the efficacy evaluation indicators, there were 6 patients with ATRX gene mutation, among the 6 patients, there were 4 patients with objective response evaluation, which was 66.7% of all patients with mutation, and there were 14 patients with wild -type ATRX gene, among the 14 patients, 0 patient had objective response evaluation, which was 0% of all wild-type patients. There was a significant statistic difference between the objective response of the patients with ATRX gene mutation and the objective response of the wild-type patients, and the P value was 0.0031. The above results indicated that the patients with ATRX gene mutation can obtain better benefit from the Chiauranib therapy.

The above are only preferred embodiments of the present invention. It should be pointed out that those skilled in the art can further make a plurality of improvements and modifications without departing from the principle of the present invention, and these improvements and modifications shall fall within the scope of protection of the present invention.

## Claims

1. Use of KDM5A gene and/or ATRX gene as biomarkers in evaluating efficacy of Chiauranib or guiding an administration of Chiauranib.

2. Use of a product for detecting KDM5A gene and/or ATRX gene mutation for the manufacture of a product for evaluating efficacy of Chiauranib or guiding an administration of Chiauranib.

3. Use of KDM5A gene and/or ATRX gene for the manufacture of a biomarker for evaluating efficacy of Chiauranib or guiding an administration of Chiauranib.

4. A method of evaluating efficacy of Chiauranib or guiding an administration of Chiauranib, **characterized by** comprising performing a gene mutation detection on a tumor tissue of small cell lung cancer, and determining efficacy of Chiauranib is better if a gene mutation occurs in KDM5A gene or ATRX gene.

5. The method according to claim 4, **characterized in that** test sample of the detection is ctDNA, tumor tissues, tumor circulating cells or tissues from other human sources.

6. The method according to claim 4, **characterized in that** a detection method of the detection is gene sequencing, PCR, FISH, immunohistochemistry, ELISA, Western, or flow cytometry.

7. Use of Chiauranib for the treatment of small cell lung cancer patients with gene mutations in KDM5A gene or ATRX gene.

8. Use of Chiauranib for the manufacture of a drug for treating small cell lung cancer patients with gene mutations in KDM5A gene or ATRX gene.
